# EUROPEAN PATENT SPECIFICATION

(11) **EP 2 041 068 B1**
(45) Date of publication and mention of the grant of the patent: **25.01.2017**
(21) Application number: 06780126.6
(22) Date of filing: 18.07.2006
(51) Int. Cl.: C07C 327/30, A61K 31/216, A61P 29/00, A61P 43/00

(54) **POSITIVELY CHARGED WATER-SOLUBLE PRODRUGS OF IBUPROFEN WITH VERY FAST SKIN PENETRATION RATE**
POSITIVGELADENE, WASSERLÖSLICHE PRODRUGS VON IBUPROFEN MIT EINER SEHR SCHNELLEN HAUTPENETRATIONSRATE
PRO-MÉDICAMENTS D'IBUPROFÈNE HYDROSOLUBLES POSITIVEMENT CHARGÉS À TAUX DE PÉNÉTRATION CUTANÉE TRÈS RAPIDE

(43) Date of publication of application: 01.04.2009
(73) Proprietor: Techfields Biochem Co. Ltd, Shanghai N/A 200444 (CN); Yu, Chongxi, Kensington, MD 20895 (US)
(72) Inventor: YU, Chongxi, Plainfield, IL 60585 (US); XU, Lina, Shanghai N/A 200444 (CN)
(74) Representative: Finnegan Europe LLP
(86) International application number: PCT/IB2006/052461
(87) International publication number: WO 2008/010025

(56) References cited:
- EP-A2- 0 237 495
- EP-A2- 0 289 262
- WO-A1-98/47502
- WO-A1-02/068377
- WO-A1-03/029187
- WO-A2-02/085297
- US-A1- 2004 022 837
- SHANBHAG, VRINDA R. ET AL: "Ester and amide prodrugs of ibuprofen and naproxen: synthesis, anti-inflammatory activity, and gastrointestinal toxicity" JOURNAL OF PHARMACEUTICAL SCIENCES, vol. 81, no. 2, 1992, pages 149-154, XP000248778 ISSN: 0022-3549
- VENUTI M C ET AL: "SYNTHESIS AND BIOLOGICAL EVALUATION OF OMEGA-(N,N,N-TRIALKYLAMMONIUM) ALKYL ESTERS AND THIOESTERS OF CARBOXYLIC ACID NONSTEROIDAL ANTIINFLAMMATORY AGENTS" PHARMACEUTICAL RESEARCH, vol. 6, no. 10, 1 January 1989 (1989-01-01), pages 867-873, XP001098334 KLUWER ACADEMIC PUBLISHERS, NEW YORK, NY, US ISSN: 0724-8741 DOI: 10.1023/A:1015960522189
- DATABASE CA [Online] CHEMICAL ABSTRACTS SERVICE, COLUMBUS, OHIO, US; 2002, SONG, NI ET AL: "Synthesis of a quaternary ammonium derivative of ibuprofen" XP002590079 retrieved from STN Database accession no. 2003:29322 -& SONG N ET AL: "Synthesis of a derivative quaternary ammonium-ibuprofen" JOURNAL OF OCEAN UNIVERSITY OF QINGDAO,, vol. 32, no. 6, 1 January 2002 (2002-01-01), pages 911-913, XP001539131 ISSN: 1001-1862
- FUNT ET AL: "Oral ibuprofen and minocycline for the treatment of resistant acne vulgaris" JOURNAL OF THE AMERICAN ACADEMY OF DERMATOLOGY, C.V. MOSBY, ST. LOUIS, MO, US LNKD- DOI:10.1016/S0190-9622(85)80375-3, vol. 13, no. 3, 1 September 1985 (1985-09-01), pages 524-525, XP025590312 ISSN: 0190-9622 [retrieved on 1985-09-01]
- TJEBBES G W A ET AL: "d-Ibuprofen in ocular inflammation induced by paracentesis of the rabbit eye" PROSTAGLANDINS, vol. 40, no. 1, 1 July 1990 (1990-07-01), pages 29-33, XP023448748 BUTTERWORTH, STONEHAM, MA, US ISSN: 0090-6980 [retrieved on 1990-07-01]
- W.F. DIVEN ET AL: "Treatment of experimental acute otitis media with ibuprofen and ampicillin" INT.J.PEDIATR.OTORHINOLARYNGOL., vol. 33, 1995, pages 127-139, XP002590081
- ALLEGRETTI M. ET AL.: '2-Arylpropionic CXC Chemokine Receptor 1 (CXCR1) Ligands as Novel Noncompetitive CXCL8 Inhibitors' JOURNAL OF MEDICINAL CHEMISTRY vol. 48, no. 13, 27 May 2005, pages 4312 - 4331, XP008102465
- BIRSEN TOZKOPARAN ET AL.: '6-Benzylidenethiazolo[3,2-b]-1,24-triazole -5(6H)-ones substituted with ibuprofen: synthesis, characterization and evaluation of anti-inflammatory activity' EUROPEAN JOURNAL OF MEDICINAL CHEMISTRY vol. 35, no. 7-8, August 2000, pages 743 - 750, XP004372928
- HACKING M.A.P.J. ET AL.: 'Lipase catalysed acylation of hydroxylamine and hydrazine derivatives' JOURNAL OF MOLECULAR CATALYSIS B: EXZYMATIC vol. 11, no. 4-6, 22 January 2001, pages 1147 - 1105, XP008102468

## Description

### Technical Field

The present invention relates to the preparations of positively charged and water-soluble pro-drugs of 2-(p-isobutylphenyl) propionic acid (ibuprofen) and their medicinal use in treating any ibuprofen-treatable conditions in humans or animals. More specifically , the present invention is to overcome the side effects that are associated with the use of ibuprofen. These pro-drugs can be administered orally or transdermally.

### Background Art

Ibuprofen is a member of the propionic acid group of nonsteroidal anti-inflammatory drugs. The synthesis of ibuprofen was originally reported in 1964 (J.S. Nicholson and S.S. Adams, Br. Patent No. 971, 700) and first used medicinally in Europe. Ibuprofen is more potent than aspirin in anti-inflammatory and prostaglandin biosynthesis inhibition assays. 'PDR Generics' (PDR Generics, 1996, second edition, Medical Economics, Montvale, New Jersey, pg 243) has listed many medical uses of ibuprofen. Ibuprofen is used for the relief of rheumatoid arthritis and osteoarthritis symptoms, the reduction of fever, and the treatment of dysmenorrhea. Ibuprofen is used alone or as an adjunct in the treatment of Bartter's syndrome and chronic uveitis, both anterior and posterior. It is also used in IUD-associated uterine bleeding, and prophylactically to reduce the severity of nausea and prevent radiation-induced vomiting in patients receiving pelvic irradiation. Ibuprofen is also prescribed for diabetic neuropathy, acute migraine headaches, and is used in hemophilic arthropathy. It is also used in the treatment of bone loss (Jee; Webster S. S. U.S. Pat. No. 5,604,259), prevention or treatment of sunburn (Sunshine: Abraham. U.S. Pat. No. 5,100,918)

Unfortunately, a number of side effects are associated with the use of ibuprofen, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of ibuprofen into the host by formulation. Susan Milosovich, et. al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin -60 times faster than does the drug (TS) itself [Susan Milosovich, et al., J. Pharm. Sci., 82, 227(1993).

### Disclosure of Invention

### Technical Problem

Ibuprofen has been used medicinally for more than 30 years and ibuprofen is more potent than aspirin in anti-inflammatory and prostaglandin biosynthesis inhibition. Ibuprofen is indicated for the relief of rheumatoid arthritis and osteoarthritis symptoms, the relief of mild to moderate pain, the reduction of fever, and the treatment of dysmenorrhea. Ibuprofen is used alone or as an adjunct in the treatment of Bartter's syndrome and chronic uveitis, both anterior and posterior. Ibuprofen is also prescribed for diabetic neuropathy, acute migraine headaches, and is used in hemophilic arthropathy. It is also used in IUD-associated uterine bleeding, and prophylactically to reduce the severity of nausea and prevent radiation-induced vomiting in patients receiving pelvic irradiation.

Unfortunately, a number of side effects are associated with the use of ibuprofen, most notably GI disturbances such as dyspepsia, heartburn, vomiting, gastroduodenal bleeding, gastric ulcerations, and gastritis. Gastroduodenal bleeding induced by ibuprofen is generally painless but can lead to fecal blood loss and may cause a persistent iron deficiency anemia.

### Technical Solution

This invention relates to the preparation of novel positively charged pro-drugs of ibuprofen and their use medicinally. These pro-drugs have the general formula (1) "Structure 1".

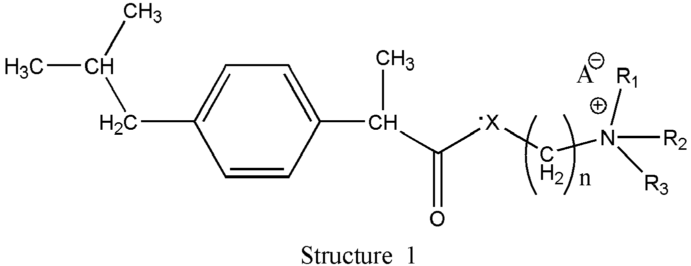

In Structure 1, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; R₃ represents H; X represents S; A represents any negative ions; and n= 0, 1, 2, 3, 4, 5, 6, 7, 8, 9 or 10.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

The goal of this invention is to avoid the side effects of ibuprofen by increasing the solubility of ibuprofen in gastric juice and the penetration rate of ibuprofen through the membrane and skin barrier which will make it administrable transdermally (topical application). These novel pro-drugs of ibuprofen have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The following examples relating to diethylaminoethyl 2-(p-isobutylphenyl) propionate.AcOH are comparative.

The solubility of diethylaminoethyl 2-(p-isobutylphenyl) propionate. AcOH and 2-(ρ-isobutylphenyl) propionic acid (ibuprofen) in water were >300 mg and 0.05 mg/ml. In many instances, the slowest or rate-limiting step in the sequence is the dissolution of the drug. Ibuprofen has a very low solubility in gastric juice. It stays in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the prodrugs will not cause gastric mucosal cell damage.

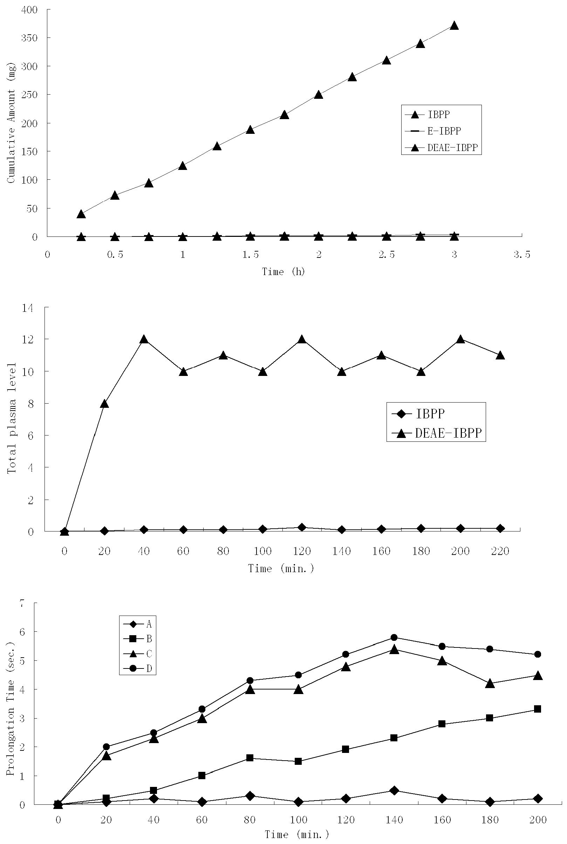

The penetration rates of diethylaminoethyl 2-(ρ-isobutylphenyl) propionate.AcOH and 2-(ρ-isobutylphenyl) propionic acid through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of 2-(ρ-isobutylphenyl) propionic acid (ibuprofen) and diethylaminoethyl 2-(ρ-isobutylphenyl) propionate.AcOH penetrating the skin versus time were determined by a specific high-performance liquid chromatography method. The results using a donor consisting of either a 30% suspension of 2-(ρ-isobutylphenyl) propionic acid (ibuprofen) and ethyl 2-(ρ-isobutylphenyl) propionate and 30% solution of diethylaminoethyl 2-(ρ-isobutylphenyl) propionate.AcOH in 2mL of pH 7.4 phosphate buffer (0.2M) are shown in Figure 1. Apparent flux values of 0.5 mg, 1 mg and 125 mg/cm²/h were calculated for ibuprofen, ethyl 2-(ρ-isobutylphenyl) propionate (the non positive charged normal ester of 2-(ρ-isobutylphenyl) propionic acid), and diethylaminoethyl 2-(ρ-isobutylphenyl) propionate. AcOH. The results suggest that the pro-drug, diethylaminoethyl 2-(ρ-isobutylphenyl) propionate.AcOH diffuses through human skin -250 times faster than does 2-(ρ-isobutylphenyl) propionic acid itself and -125 times faster than does ethyl 2-(ρ-isobutylphenyl) propionate. The normal ester, ethyl 2-(ρ-isobutylphenyl) propionate and 2-(ρ-isobutylphenyl) propionic acid itself have very similar penetration rates (only 2 times difference). The results suggest that the positive charge on the dialkyaminoethyl group has a very important role in the passage of the drug across the membrane and skin barrier. Other prodrugs of the general "Structure 1" have very high penetration rates and are very close to that of diethylaminoethyl 2-(ρ-isobutylphenyl) propionate.AcOH.

The in vivo rates of penetration of 2-(ρ-isobutylphenyl) propionic acid (IBPP) and diethylaminoethyl 2-(ρ-isobutylphenyl) propionate.AcOH (DEAE-IBPP) through the skin of intact hairless mice were compared. The donor consisted of either a 30% solution of 2-(ρ-isobutylphenyl) propionic acid (IBPP) or diethylaminoethyl 2-(ρ-isobutylphenyl) propionate.AcOH (DEAE-IBPP) in 1 mL of isopropanol applied to a 1 cm² on the backs of the hairless mice. Plasma levels of ibuprofen and diethylaminoethyl 2-(ρ-isobutylphenyl) propionate were determined by a specific high-performance liquid chromatography method. The results (Figure 2) show that the peak levels of diethylaminoethyl 2-(ρ-isobutylphenyl) propionate were reached -30 minutes after application of the donor systems. It takes 1-2 hours for ibuprofen to reach the peak ibuprofen level when it is taken orally. The peaks were -0.2 mg/ml for 2-(ρ-isobutylphenyl) propionic acid (ibuprofen) or -12 mg/ml for diethylaminoethyl 2-(ρ-isobutylphenyl) propionate.AcOH (approximately 60 times difference). -12 mg/ ml of 2-(ρ-isobutylphenyl) propionic acid in plasma is more than 30 times higher than the 2-(ρ-isobutylphenyl) propionic acid plasma level for effective analgesia and effective antiinflammatory activity. This is a very exciting result. It will be very easy and fast to deliver therapeutically effective plasma levels of 2-(ρ-isobutylphenyl) propionic acid into the host by these pro-drugs. These results suggest that the pro-drugs can be administered not only orally, but also transdermally for any kind of medical treatments. The in vivo rates of penetration of other Pro-drugs of the general "Structure 1" are close to that of diethylaminoethyl 2-(ρ-isobutylphenyl) propionate.AcOH .

To check the gastroduodenal bleeding caused by drugs, rats (two groups, each group had 10 rats) were orally administered with 100 mg/kg of ibuprofen or diethylaminoethyl 2-(ρ-isobutylphenyl) propionate.AcOH per day for 21 days. We found an average of 4 mg of fecal blood per gram of feces in the ibuprofen group and none in diethylaminoethyl 2-(ρ-isobutylphenyl) propionate.AcOH group.

Ibuprofen has demonstrated anti-inflammatory, analgesic, antipyretic, and antirheumatic activity. A good prodrug should go back to the drug itself in plasma. Diethylaminoethyl ester group of diethylaminoethyl 2-(ρ-isobutylphenyl) propionate.AcOH can be rapidly cleaved by the enzymes in human plasma in vitro and more than 90% of the pro-drug is changed back to ibuprofen. Due to the pro-drug having a much better absorption rate, the prodrug will have more strength than ibuprofen itself at the same dosage. The analgetic, antipyretic, and anti-inflammatory activities of diethylaminoethyl 2-(ρ-isobutylphenyl) propionate.AcOH were tested using ibuprofen as a comparison. Other compounds of the general 'Structure 1' were tested by the same methods and have very similar results as that of diethylaminoethyl 2-(ρ-isobutylphenyl) propionate.AcOH.

Analgetic activity: The prolongation time of pain the threshold of a mouse tail was determined in accordance with the D'Amour-Smith Method (J. Pharmacol. Exp. Ther., 72, 74(1941)). After 200mg/kg of ibuprofen was administered orally and 200mg/kg of diethylaminoethyl 2-(ρ-isobutylphenyl) propionate.AcOH was administered orally and transdermally, the tails of mice were exposed to heat and the prolongation time of pain threshold was determined. The results obtained are shown in Figure 3. The groups administered 200 mg/kg of diethylaminoethyl 2-(ρ-isobutylphenyl) propionate.AcOH orally (C) and transdermally (D) were shown to exhibit stronger analgetic activity than the group administered 200 mg/kg of ibuprofen.

The number of writhings that occurred when mice were administered an acetic acid solution intraperitoneally were counted, and the rate of inhibition based on the control group was calculated. 42 mice were divided into 7 groups (6 mice each). Ibuprofen (IBPP, 50 mg/kg and 100 mg) was administered to groups B1 and B2 of mice and diethylaminoethyl 2-(ρ-isobutylphenyl) propionate.AcOH (DEAE-IBPP, 50 mg and 100 mg/kg) was administered orally to groups C1 and C2. Diethylaminoethyl 2-(ρ-isobutylphenyl) propionate.AcOH (DEAE-IBPP, 50 mg and 100 mg/kg) was administered transdermally to groups D1 and D2. The A group is the control group. The test compounds were administered to the mice 30 minutes before the acetic acid solution was administered. The results are shown in Table 1.

**Table 1. Te rate of writhings inhibition by and ibuprofen and its pro-drugs**

| Group | A | B1 | B2 | C1 | C2 | D1 | D2 |
|---|---|---|---|---|---|---|---|
| Dose (mg/kg) | 0 | 50 | 100 | 50 | 100 | 50 | 100 |
| No. of writhings | 34.2 | 17.2 | 13.1 | 14.1 | 10.2 | 13.3 | 9.8 |
| % | - | 50 | 62 | 59 | 70 | 61 | 71 |

The results show that diethylaminoethyl 2-(ρ-isobutylphenyl) propionate.AcOH demonstrates better analgetic activity than ibuprofen. Other compounds of the general 'Structure 1' show similar analgetic activity.

Antipyretic activity: Rats received a sterilized E. coli suspension as a pyrogen. 56 rats were divided into 7 groups. The control group is group A. 2 hours later, ibuprofen (IBPP, B1 for 100mg/kg and B2 for 150mg/kg) and diethylaminoethyl 2-(ρ-isobutylphenyl) propionate.AcOH (DEAE-IBPP, C1 for 100mg/kg and C2 for 150 mg) were administered orally and diethylaminoethyl 2-(ρ-isobutylphenyl) propionate.AcOH (DEAE-IBPP, D1 for 100 mg and D2 for 150 mg/kg) was administered transdermally. The body temperatures of the rats were taken at 90 min. intervals before and after the administration of the test compounds. The results are shown in Table 2.

**Table 2. Antipyretic activity of ibuprofen and its pro-drugs**

| Compound | t=0 min. | t=90 min. | t=180 min. | t=270 min. |
|---|---|---|---|---|
| Control group(A) | 37.35±0.05 | 37.25±0.07 | 37.33±0.05 | 37.32±0.08 |
| IBPP (100mg/kg, B1) | 37.25±0.06 | 36.83±0.05 | 36.80±0.08 | 36.78±0.07 |
| IBPP (150mg/kg, B2) | 37.35±0.09 | 36.59±0.07 | 36.53±0.06 | 36.55±0.05 |
| DEAE-IBPP (100mg/kg, C1, orally) | 37.22±0.07 | 36.40±0.06 | 36.50±0.05 | 36.45±0.08 |
| DEAE-IBPP (150mg/kg, C2, orally) | 37.24±0.08 | 36.30±0.05 | 36.35±0.07 | 36.38±0.08 |
| DEAE-IBPP (100mg/kg, D1, transdermally) | 37.27±0.06 | 36.30±0.06 | 36.35±0.08 | 36.31±0.07 |
| DEAE-IBPP (150mg/kg, D2, transdermally) | 37.26±0.05 | 36.25±0.05 | 36.30±0.07 | 36.20±0.05 |

The results shown that diethylaminoethyl 2-(p-isobutylphenyl) propionate.AcOH demonstrated antipyretic activity at 100 mg/kg dose and better than ibuprofen. The results show that transdermal administration of diethylaminoethyl 2-(p-isobutylphenyl) propionate.AcOH is better than oral administration. Other compounds of the general "Structure 1" show similar antipyretic activity.

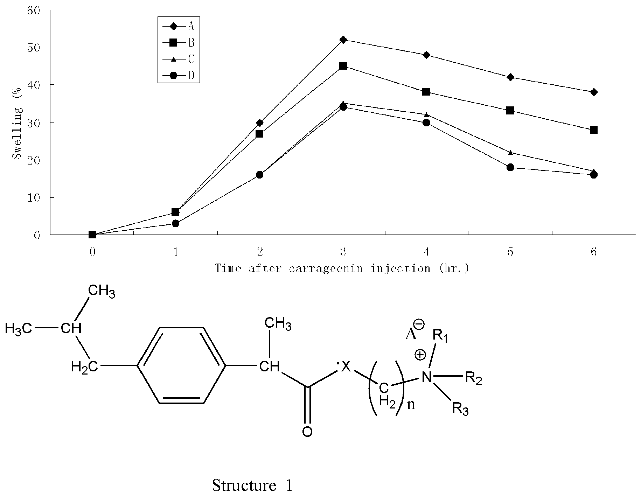

Anti-inflammatory activity: 50 mg/kg of diethylaminoethyl 2-(ρ-isobutylphenyl) propionate.AcOH was administered orally or transdermally to rats and 50 mg/kg of ibuprofen was administered orally. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that diethylaminoethyl 2-(p-isobutylphenyl) propionate.AcOH by oral administration and transdermal administration demonstrated better Anti-inflammatory activity than that of ibuprofen at 50mg/kg by oral administration. Other compounds of the general 'Structure 1' show similar anti-inflammatory activity.

It is also known that a high dose of oral ibuprofen shows an anti-reactive-antiasthmatic activity by inhibition of the cyclooxygenase activity. Due to their very high membrane penetration rate, these prodrugs can be used in treating asthma by spraying into the mouth or nose of the host. They can also be used to treat acne due to their anti-inflammatory properties.

The compounds of the general formula (1) "Structure 1" indicated above can be prepared from ibuprofen or from functional derivatives of ibuprofen, for example, acid halides or mixed anhydrides of the general formula (2) 'Structure 2'.

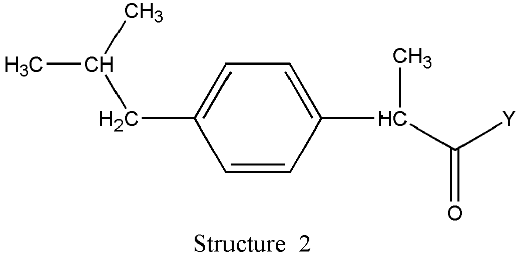

In structure 2, Y represents halogen, alkoxycarbonyl or substituted aryloxy-carbonyloxy, by reaction with compounds of the general formula (3) "Structure 3". 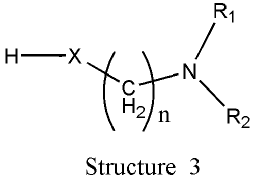 in Structure 3, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; X represents S; and n=0, 1, 2, 3, 4, 5, 6, 7, 8, 9 or 10.

The compounds of the general formula (1) "Structure 1" indicated above can be prepared from ibuprofen, by reaction with compounds of the general formula (3) "Structure 3" by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

The present invention relates to pharmaceutical preparations comprising of prodrugs of ibuprofen of the general "Structure 1" in addition to customary auxiliaries and excipients, e.g. in the form of tablets, capsules or solutions for administration orally and in the form of solutions, lotion, ointment, emulsion or gel for administration transdermally. The new active compounds of the general "Structure 1" can be combined with vitamins such as A, B, C or E or beta-carotene, or other pharmaceuticals, such as folic acid, etc. for use in treating any ibuprofen-treatable conditions in humans or animals.

It is also known that ibuprofen shows an anti-reactive-antiasthmatic activity by inhibition of the cyclooxygenase activity. Due to their very high membrane penetration rate, these pro-drugs can be used in treating asthma by spraying into the mouth or nose of the host.

They can also be used to treat acne and other skin disorders due to their anti-inflammatory properties. They can be used for the treatment and prevention of endothelia dysfunction as well.

These pro-drugs are water-soluble neutral salt and can be tolerated very well by the eye. They can be used for treating eye inflammatory diseases, for treating of ocular pain after corneal surgery, for treating glaucoma or for treating ear inflammatory and/ or painful conditions (otitis).

Transdermal therapeutic application systems of compounds of the general "Structure 1" or a composition comprising at least one compound of the general "Structure 1 ", as an active ingredient, can be used for treating any ibuprofen-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables ibuprofen to reach constantly optimal therapeutic blood levels to increase effectiveness and reduce the side effects of ibuprofen. These systems can be worn on the wrist, ankle, arm, leg, or any part of body.

### Advantageous Effects

These pro-drugs of ibuprofen have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these prodrugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drug was changed back to the drug itself. The pro-drugs have a much better absorption rate, and thus the pro-drugs will have better strength than the ibuprofen itself at the same dosage. The experiment results suggest that the pro-drug, diethylaminoethyl 2-(ρ-isobutylphenyl) propionate.AcOH, diffuses through human skin -250 times faster than ibuprofen itself and -125 times faster than ethyl 2-(ρ-isobutylphenyl) propionate. The in vivo rates of penetration of diethylaminoethyl 2-(ρ-isobutylphenyl) propionate.AcOH through the skin of intact hairless mice were very high. It takes 1-2 hours for ibuprofen tablets to reach the peak ibuprofen plasma level when it is taken orally, but diethylaminoethyl 2-(ρ-isobutylphenyl) propionate.AcOH only took about 30 minutes to reach the peak ibuprofen plasma level. The most exciting result is that the pro-drugs can be administered not only orally, but also transdermally for any type of medical treatment and should avoid most of the side effects of ibuprofen, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of 2-(p-isobutylphenyl) propionic acid (IBPP), ethyl 2- (p-isobutylphenyl) propionate (E-IBPP), and diethylaminoethyl 2-(p-isobutylphenyl) propionate. AcOH (DEAE-IBPP) crossing isolated human skin tissue in Franz cells (n=5). IBPP and E-IBPP were applied as a 30% suspension. DEAE-IBPP was applied as 30% solution. In each case, the vehicle was pH 7.4 phosphate buffer (0.2 M).
Figure 2: Total plasma levels of 2-(p-isobutylphenyl) propionic acid (IBPP) after topical application of 1 ml of a 30% solution of 2-(ρ-isobutylphenyl) propionic acid (IBPP) or diethylaminoethyl 2-(ρ-isobutylphenyl) propionate.AcOH (DEAE-IBPP) to the backs of hairless mice (n=5).
Figure 3: The prolongation time of the pain threshold of mice tails after 200 mg/kg of ibuprofen (B) was administered orally, 200 mg/kg of diethylaminoethyl 2-(ρ-isobutylphenyl) propionate.AcOH was administered orally (C) and transdermally (D). A is the control group.
Figure 4. The rate of swelling (%) after a carrageenin injection. 1 hour before the carrageenin injection, 50 mg/kg of ibuprofen was administered orally (B), 50 mg/kg of diethylaminoethyl 2-(ρ-isobutylphenyl) propionate.AcOH was administered orally (C), and transdermally (D). A is the control group.

Structure 1: R1 represents H, one of any alkyl, alkyloxy, alkenyl, or alkynyl residues having up to 12 carbon atoms, or aryl residues; R2 represents H, one of any alkyl, alkyloxy, alkenyl or alkynyl residues having up to 12 carbon atoms, or aryl residues; R3 represents H; X represents S; A-represents any negative ions; and n= 0, 1, 2, 3, 4, 5, 6, 7, 8, 9 or 10.

### Best Mode

### Comparative: Preparation of diethylaminoethyl 2-(p-isobutylphenyl) propionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 11.7 g of diethylaminoethanol were added into the reaction mixture. The mixture is stirred for 3 hours at RT. The solid side product was removed by filtration and washed with chloroform (3 x 30 ml). 6 g of acetic acid is added into the chloroform solution with stirring. The organic solution was evaporated off. After drying, it yielded 35 g of the desired product (92%). Hygroscopic product; Solubility in water: 400 mg/ml; Elementary analysis: C₂₁H₃₅NO₄; MW: 365.51. Calculated % C: 69.01; H: 9.65; N: 3.83; O: 17.51; Found % C: 68.98; H: 9.68; N: 3.82; O: 17.52. ¹H-NMR (400 MHz, CDCl₃): δ: 1.10 (d, 6H), 1.52 (d, 3H), 1.56 (t, 6H), 2.21 (s, 3H), 2.22 (m, 1H); 2.51 (d, 2H), 3.28 (m, 4H), 3.52 (m, 2H), 3.78 (m, 1H), 4.52 (t, 2H), 6.8 (b, 1H), 7.06 (d, 2H), 7.07 (d, 2H).

### Mode for Invention

### Comparative: of dimethylaminoethyl 2-(ρ-isobutylphenyl) propionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 8.9 g of dimethylaminoethanol were added into the reaction mixture. The mixture is stirred for 3 hours at RT. 6 g of acetic acid is added into the reaction mixture with stirring. The solid side product was removed by filtration and washed with chloroform (3 x 30 ml). The organic solution was evaporated off. After drying, it yielded 31 g of the desired product (92%). Hygroscopic product; Solubility in water: 400 mg/ml; Elementary analysis: C₁₉H₃₁NO₄; MW: 337.45. Calculated % C: 67.63; H: 9.26; N: 4.15; O: 18.96; Found % C: 67.60; H: 7.28; N: 4.14; O: 18.98. ¹H-NMR (400 MHz, CDCl₃): δ: 1.01 (d, 6H), 1.52 (d, 3H), 2.21 (s, 3H), 2.22 (m, 1H); 2.51 (d, 2H), 2.90 (s, 6H), 3.52 (m, 2H), 3.78 (m, 1H), 4.52 (t, 2H), 6.8 (b, 1H), 7.06 (d, 2H), 7.07 (d, 2H).

### Preparation of S-dimethylaminoethyl 2-(p-isobutylphenyl)thiopropionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 9.3 g of dimethylaminoethyl mercaptan were added into the reaction mixture. The mixture was stirred for 3 hours at RT. 6 g of acetic acid was added into the reaction mixture with stirring. The solid side product was removed by filtration and washed with chloroform (3 x 30 ml). The organic solution was evaporated off. After drying, it yielded 32 g of the desired product (90.5 %). Hygroscopic product; Solubility in water: 300 mg/ml; Elementary analysis: C₁₉H₃₁NO₃S; MW: 353.52. Calculated % C: 64.55; H: 8.84; N: 3.96; O: 13.58, S: 9.07; Found % C: 64.52; H: 8.86; N: 3.95; O: 13.62; S: 9.05. ¹H-NMR (400 MHz, CDCl₃): δ: 1.01 (d, 6H), 1.52 (d, 3H), 2.20 (s, 3H), 2.22 (m, 1H); 2.50 (d, 2H), 2.90 (s, 6H), 3.31 (t, 2H), 3.81 (t, 1H), 3.91 (t, 2H), 6.8 (b, 1H), 7.06 (d, 2H), 7.07 (d, 2H).

### Comparative: Preparation of N-dimethylaminoethyl 2-(p-isobutyliphenyl)propionamide.AcOH

22.5 g (0.1 mol) of 2-(ρ-isobutylphenyl) propionyl chloride was dissolved in 100 ml of chloroform. The mixture was cooled to 0°C. 15 ml of triethylamine and 8.9 g of dimethylaminoethylamine were added into the reaction mixture. The mixture was stirred for 3 hours at RT. 6 g of acetic acid was added into the reaction mixture with stirring. The solid side product was removed by filtration and washed with chloroform (3 x 30 ml). The organic solution was evaporated off. After drying, yielded 30 g of the desired product (89.1 %). Hygroscopic product; Solubility in water: 300 mg/ml; Elementary analysis: C₁₉H₃₂N₂O₃; MW: 336.47. Calculated % C: 67.82; H: 9.59; N: 8.33; O: 14.27; Found % C: 67.80; H: 9.61; N: 8.31; O: 14.26. ¹H-NMR (400 MHz, CDCl₃): δ: 1.01 (d, 6H), 1.52 (d, 3H), 2.20 (s, 3H), 2.22 (m, 1H); 2.50 (d, 2H), 2.90 (s, 6H), 3.50(t, 2H), 3.64 (t, 2H), 3.89 (m, 1H), 6.8 (b, 1H), 7.06 (d, 2H), 7.07 (d, 2H), 7.8 (b, 1H).

### Preparation of S-diethylaminoethyl 2-(p-isobutylphenyl)thiopropionate.AcOH

20.6 g (0.1 mol) of 2-(ρ-isobutylphenyl) propionic acid was dissolved in 100 ml of dichloromethane (DCM). The mixture was cooled to 0°C. 20.6 g of 1,3-Dicyclohexylcarbodiimid was added into the reaction mixture. The mixture was stirred for 30 minutes at 0°C. 13.4 g (0.1 mol) of diethylaminoethyl mercaptan was added into the reaction mixture. The mixture was stirred for 3 h at RT. 6 g of acetic acid was added into the reaction mixture with stirring. The solid side product was rem oved by filtration and washed with chloroform (3 x 50 ml). The organic solution was evaporated off. After drying, it yielded 34 g of the desired product (89.1 %). Hygroscopic product; Solubility in water: 300 mg/ml; Elementary analysis: C₂₁H₃₅NO₃S; MW: 381.57. Calculated % C: 66.10; H: 9.25; N: 3.67; O: 12.58, S: 8.40; Found % C: 66.07; H: 9.29; N: 3.66; O: 12.60; S: 8.38. ¹H-NMR (400 MHz, CDCl₃): δ: 1.01 (d, 6H), 1.52 (d, 3H), 1.56 (t, 6H) 2.20 (s, 3H), 2.22 (m, 1H); 2.50 (d, 2H), 3.26 (m, 4H), 3.31 (t, 2H), 3.81 (t, 1H), 3.91 (t, 2H), 6.8 (b, 1H), 7.06 (d, 2H), 7.07 (d, 2H).

### Comparative: Preparation of N-dimethylaminopropyl 2-(p-isobutylphenyl)propionamide.AcOH

20.6 g (0.1 mol) of 2-(ρ-isobutylphenyl) propionic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 13.1 g of dimethylaminopropylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 32 g of the desired product (91.2%). Hygroscopic product; Solubility in water: 320 mg/ml; Elementary analysis: C₂₀H₃₄N₂O₃; MW: 350.5. Calculated % C: 68.54; H: 9.78; N: 7.99; O: 13.69; Found % C: 68.51; H: 9.80; N: 7.98; O: 13.71. ¹H-NMR (400 MHz, CDCl₃): δ: 1.01 (d, 6H), 1.52 (d, 3H), 1.98 (m, 2H), 2.20 (s, 3H), 2.22 (m, 1H); 2.50 (d, 2H), 2.90 (s, 6H), 3.20(m, 2H), 3.24 (m, 2H), 3.89 (m, 1H), 6.8 (b, 1H), 7.06 (d, 2H), 7.07 (d, 2H), 7.8 (b, 1H).

### Comparative: Preparation of dipropylaminoethyl 2-(p-isobutylphenyl)propionate.AcOH

22.3 g (0.1 mol) of sodium 2-(ρ-isobutylphenyl) propionate was suspended in 180 ml of chloroform. 28.8 g (0.1 mol) of dipropylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 35 g of the desired product (88.9%). Hygroscopic product; Solubility in water: 300 mg/ml; Elementary analysis: C₂₃H₃₉NO₄; MW: 393.56. Calculated % C: 70.19; H: 9.99; N: 3.56; O: 16.26; Found % C: 70.14; H: 10.03; N: 3.55; O: 16.28. ¹H-NMR (400 MHz, CDCl₃): δ: 0.96 (d, 6H), δ: 1.10 (d, 6H), 1.52 (d, 3H), 1.77 (m, 4H), 2.21 (s, 3H), 2.22 (m, 1H); 2.51 (d, 2H), 3.24 (m, 4H), 3.52 (m, 2H), 3.78 (m, 1H), 4.52 (t, 2H), 6.8 (b, 1H), 7.06 (d, 2H), 7.07 (d, 2H).

### Comparative: Preparation of dipropylaminoethyl 2-(p-isobutylphenyl)propionate.AcOH

60 g of Polymer-bound triethylamine (3 mmol/g, 100-200 mesh) was suspended in 180 ml of chloroform. 20.6 g (0.1 mol) of 2-(ρ-isobutylphenyl) propionic acid was added into the mixture with stirring. 43 g (0.15mol) of dipropylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 36 g of the desired product (91.5%). Hygroscopic product; Solubility in water: 350 mg/ml; Elementary analysis: C₂₃H₃₉NO₄; MW: 393.56. Calculated % C: 70.19; H: 9.99; N: 3.56; O: 16.26; Found % C: 70.14; H: 10.03; N: 3.55; O: 16.28. ¹H-NMR (400 MHz, CDCl₃): δ: 0.96 (d, 6H), δ: 1.10 (d, 6H), 1.52 (d, 3H), 1.77 (m, 4H), 2.21 (s, 3H), 2.22 (m, 1H); 2.51 (d, 2H), 3.24 (m, 4H), 3.52 (m, 2H), 3.78 (m, 1H), 4.52 (t, 2H), 6.8 (b, 1H), 7.06 (d, 2H), 7.07 (d, 2H).

### Industrial Applicability

The pro-drugs of the general formula (1) "Structure 1" are superior to ibuprofen. They may be used medicinally in treating any ibuprofen-treatable conditions in humans or animals. They may be used for the relief of signs and symptoms of rheumatoid arthritis and osteoarthritis, the reduction of fever, and the treatment of dysmenorrhea. They are used alone or as an adjunct in the treatment of Bartter's syndrome and chronic uveitis, both anterior and posterior. It is also used in IUD-associated uterine bleeding, and prophylactically to reduce the severity of nausea and prevent radiation-induced vomiting in patients receiving pelvic irradiation. They are also prescribed for diabetic neuropathy and acute migraine headaches and are used in hemophilic arthropathy. They are also used in treatment of bone loss, prevention or treatment of sunburn. They may be used in the prevention of cancer. Due to their very high membrane penetration rate, these pro-drugs can be used in treating asthma by inhalation to a host. They can be used to treat acne due to their anti-inflammatory properties. These pro-drugs are water-soluble neutral salt and can be tolerated very well by the eye. They can be used for treating eye inflammatory diseases, for treating of ocular pain after corneal surgery, for treating glaucoma or for treating ear inflammatory and/or painful conditions (otitis).

## Claims

1. Compounds of Structure 1 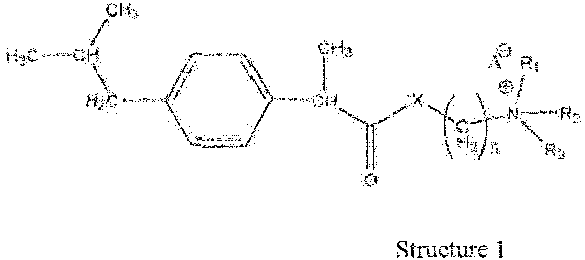 In Structure 1, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; R₃ represents H; X represents S; A- represents any negative ions; and n= 0, 1, 2, 3, 4, 5, 6, 7, 8, 9 or 10.

2. Process for the preparation of compounds according to Claim 1, wherein acid halides or mixed anhydrides of Structure 2 are reacted with compounds of Structure 3, wherein 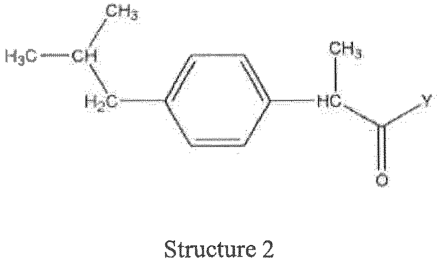 in Structure 2, Y represents halogen, alkoxycarbonyl or substituted aryloxy-carbonyloxy, and wherein 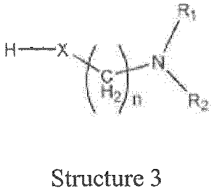 in Structure 3, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; X represents S; and n=0, 1, 2, 3, 4, 5, 6, 7, 8, 9 or 10.

3. Process for the preparation of compounds according to Claim 1, wherein the compounds are prepared from ibuprofen by reaction with compounds of Structure 3 by using coupling reagents, wherein 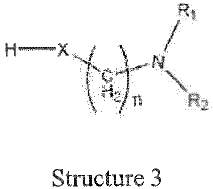 in Structure 3, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; X represents S; and n=0, 1, 2, 3, 4, 5, 6, 7, 8, 9 or 10, and the coupling ragent is selected from N,N'-Dicyclohexylcarbodiimide, N, N'-Diisopropylcarbodiimide, O-(Benzotriazol-1 -yl)-N,N,N',N'-tetramethyluronium tetrafluoroborate, O-(Benzotriazol- 1 -yl)-N,N,N',N'-tetramethyluronium hexafluo- rophosphate and Benzotriazol-1-yl-oxy-tris (dimethylamino)phosphonium hexafluo- rophosphate.

4. The compound according to claim 1 or a composition comprising at least one compound according to claim 1 as an active ingredient for use in the treatment of any ibuprofen-treatable conditions in humans or animals, wherein the compound or composition is administered orally, wherein the ibuprofen-treatable conditions are selected from the group including toothache, headache, acute migraine headache, pain of arthritis, inflammatory pain, dysmenorrhea, fever, cancer, Bartter's syndrome, anterior and posterior chronic uveitis, IUD-associated uterine bleeding, nausea, radiation-induced vomiting, diabetic neuropathy, hemophilic arthropathy, bone loss, and sunburn.

5. Compounds of Structure 1 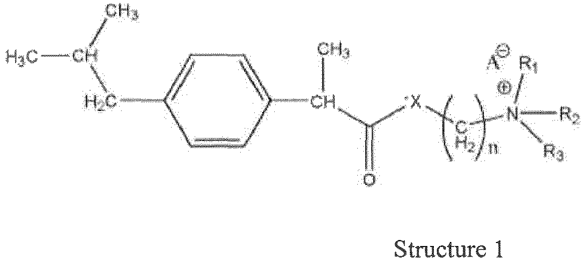 In Structure 1, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; R₃ represents H; X represents O or S; A⁻ represents any negative ions; and n= 0, 1, 2, 3, 4, 5, 6, 7, 8, 9 or 10, or a composition comprising at least one compound of Structure 1 as an active ingredient for use in the treatment of any ibuprofen-treatable conditions in humans or animals, wherein the compound or composition is administered transdermally, wherein the ibuprofen-treatable conditions are selected from the group including toothache, headache, acute migraine headache, pain of arthritis, inflammatory pain, dysmenorrhea, fever, cancer, Bartter's syndrome, anterior and posterior chronic uveitis, IUD-associated uterine bleeding, nausea, radiation-induced vomiting, diabetic neuropathy, hemophilic arthropathy, bone loss, and sunburn.

6. The compound or composition for use according to claim 5, wherein the compound or composition is in the form of a solution, spray, lotion, ointment, emulsion or gel, and administered transdermally to any part of body to deliver therapeutically effective plasma levels.

7. The compound according to claim 1 or a composition comprising at least one compound according to claim 1 as an active ingredient for use in the treatment of pain, wherein the compound or composition is topically administered to the inflamed area in a therapeutically effective amount.

8. The compound or composition for use according to claim 7, wherein the pain is headache, toothache, and muscle pain, and arthritis and other inflammatory pain.

9. The compound according to claim 1 or a composition comprising at least one compound according to claim 1 as an active ingredient for use in the treatment of acne, sunburn or other skin disorders, and wherein the compound or composition is in the form of a solution, spray, lotion, ointment, emulsion or gel.

10. Compounds of Structure 1 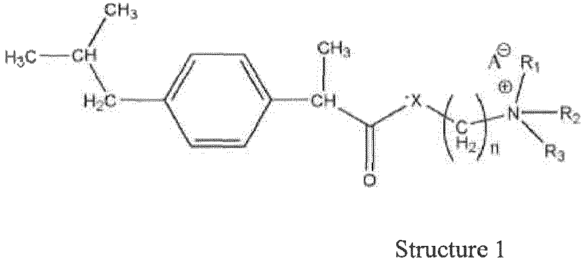 In Structure 1, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; R₃ represents H; X represents O or S; A⁻ represents any negative ions; and n= 0, 1, 2, 3, 4, 5, 6, 7, 8, 9 or 10, or a composition comprising at least one compound of Structure 1 as an active ingredient for use in the treatment of acne, sunburn or other skin disorders, wherein the compound or composition is in the form of a solution, spray, lotion, ointment, emulsion or gel, and wherein compound or composition is administered transdermally.

11. The compound according to claim 1 or a composition comprising at least one compound according to claim 1 as an active ingredient for use in the treatment of asthma, wherein the compound or composition is administered by spraying to through the mouth or nose or other parts of body.

12. The compound according to claim 1 or a composition comprising at least one compound according to claim 1 as an active ingredient for use in the treatment of any eye inflammatory diseases, ocular pain after corneal surgery, glaucoma or ear inflammatory and/or painful conditions in humans or animals.

13. The compound or composition for use according to claim 12, wherein the ear inflammatory and/or painful condition is otitis.

14. Transdermal therapeutic application systems of Compounds of Structure 1 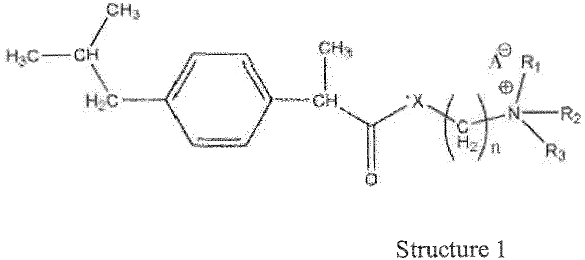 In Structure 1, R₁ represents H, one of any alkyl, alkyloxy, alkenyl, or alkynyl residues having up to 12 carbon atoms, or aryl residues; R₂ represents H, one of any alkyl, alkyloxy, alkenyl or alkynyl residues having up to 12 carbon atoms, or aryl residues; R₃ represents H; X represents O or S; A⁻ represents any negative ions; and n= 0, 1, 2, 3, 4, 5, 6, 7, 8, 9 or 10, or a composition comprising at least one compound of Structure 1 as an active ingredient for use in the treatment of any ibuprofen-treatable conditions in humans or animals, wherein transdermal therapeutic application systems enable the ibuprofen to reach constantly optimal therapeutic blood levels to increase effectiveness and reduce the side effects of ibuprofen wherein the ibuprofen-treatable conditions are selected from the group including toothache, headache, acute migraine headache, pain of arthritis, inflammatory pain, dysmenorrhea, fever, cancer, Bartter's syndrome, anterior and posterior chronic uveitis, IUD-associated uterine bleeding, nausea, radiation-induced vomiting, diabetic neuropathy, hemophilic arthropathy, bone loss, and sunburn.

15. The transdermal therapeutic application systems according to claim 14, **characterized in that** these systems are a bandage or a patch comprising of one active substance-containing matrix layer and an impermeable backing layer.

16. The transdermal therapeutic application systems according to claims 14-15, **characterized in that** the system has an active substance reservoir, which has a permeable bottom facing the skin.

## Patentansprüche

1. Verbindungen der Struktur 1 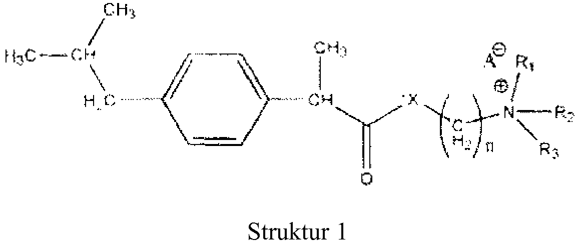 In der Struktur 1 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einem von jeden Alkyl-, Alkyloxy-, Alkenyl- oder Alkynyl-Resten mit bis zu 12 Kohlenstoffatomen, oder Aryl-Reste; R₃ entspricht H; X entspricht S; A⁻ entspricht allen negativen Ionen; und n= 0, 1, 2, 3, 4, 5, 6, 7, 8, 9 oder 10.

2. Prozess zur Herstellung von Verbindungen nach Anspruch 1, wobei Säurehalide oder gemischte Anhydride der Struktur 2 mit Verbindungen der Struktur 3 reagiert werden, wobei 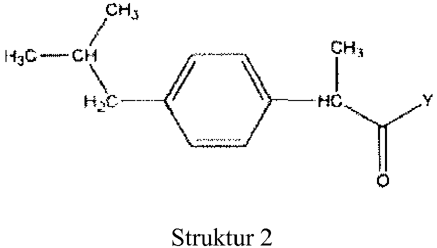 in der Struktur 2 Y Halogen, Alkoxycarbonyl oder ersetztes Aryloxy-Carbonyloxy entspricht, und wobei 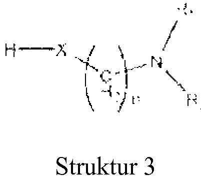 in der Struktur 3 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einem von jeden Alkyl-, Alkyloxy-, Alkenyl- oder Alkynyl-Resten mit bis zu 12 Kohlenstoffatomen, oder Aryl-Resten; X entspricht S; und n= 0, 1, 2, 3, 4, 5, 6, 7, 8, 9 oder 10.

3. Prozess zur Herstellung von Verbindungen nach Anspruch 1, wobei die Verbindungen aus Ibuprofen durch Reaktion mit Verbindungen der Struktur 3 unter Verwendung von Haftvermittlern zubereitet werden, wobei 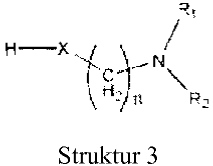 in der Struktur 3 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einem von jeden Alkyl-, Alkyloxy-, Alkenyl- oder Alkynyl-Resten mit bis zu 12 Kohlenstoffatomen, oder Aryl-Resten; X entspricht S; und n= 0, 1, 2, 3, 4, 5, 6, 7, 8, 9 oder 10, und der Haftvermittler ist ausgewählt von N,N'-Dicyclohexylcarbodiimid, N,N'-Diisopropylcarbodiimid, O-(Benzotriazol-1-yl)-N,N,N',N'-Tetramethyluronium-tetrafluorborat, O-(Benzotriazol-1-yl)-N,N,N',N'-Tetramethyluromum-hexafluorphosphat und Benzotriazol-1-yl-oxy-tris(dimethylamino)phosphonium-hexafluorphosphat.

4. Verbindung nach Anspruch 1 oder eine Zusammensetzung umfassend mindestens eine Verbindung nach Anspruch 1 als Wirkstoff zur Verwendung in der Behandlung von allen mit Ibuprofen behandelbaren Zuständen in Menschen oder Tieren, wobei der Verbindung oder Zusammensetzung oral verabreicht wird, wobei die mit Ibuprofen behandelbaren Zustände ausgewählt sind von der Gruppe einschließlich Zahnschmerzen, Kopfschmerzen, akute Migränekopfschmerzen, Schmerzen durch Arthritis, Entzündungsschmerzen, Dysmenorrhö, Fieber, Krebs, Bartter-Syndrom, vor und nach chronischer Uveitis, mit IUD verbundener uteriner Blutung, Übelkeit, durch Bestrahlung hervorgerufenes Erbrechen, diabetische Neuropathie, homophile Arthropathie, Knochenschwund und Sonnenbrand.

5. Verbindungen der Struktur 1 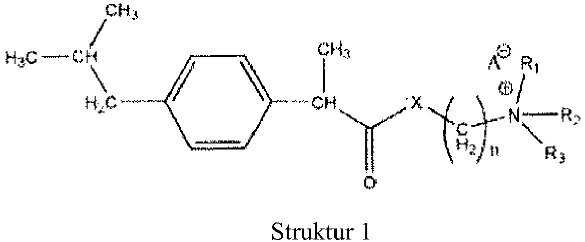 In der Struktur 1 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einem von jeden Alkyl-, Alkyloxy-, Alkenyl- oder Alkynyl-Resten mit bis zu 12 Kohlenstoffatomen, oder Aryl-Reste; R₃ entspricht H; X entspricht O oder S; A⁻ entspricht allen negativen Ionen; und n= 0, 1, 2, 3, 4, 5, 6, 7, 8, 9 oder 10, oder eine Zusammensetzung umfassend mindestens eine Verbindung der Struktur 1 als Wirkstoff zur Verwendung in der Behandlung von allen mit Ibuprofen behandelbaren Zuständen in Menschen oder Tieren, wobei der Verbindung oder Zusammensetzung transdermal verabreicht wird, wobei die mit Ibuprofen behandelbaren Zustände ausgewählt sind von der Gruppe einschließlich Zahnschmerzen, Kopfschmerzen, akute Migränekopfschmerzen, Schmerzen durch Arthritis, Entzündungsschmerzen, Dysmenorrhö, Fieber, Krebs, Bartter-Syndrom, vor und nach chronischer Uveitis, mit IUD verbundener uteriner Blutung, Übelkeit, durch Bestrahlung hervorgerufenes Erbrechen, diabetische Neuropathie, homophile Arthropathie, Knochenschwund und Sonnenbrand.

6. Verbindung oder Zusammensetzung zur Verwendung nach Anspruch 5, wobei die Verbindung oder Zusammensetzung in der Form einer Lösung, eines Sprays, einer Lotion, Salbe, Emulsion oder eines Gels ist und transdermal an einen Körperteil verabreicht wird, um therapeutisch wirksame Plasmawerte zuzuführen.

7. Verbindung nach Anspruch 1 oder eine Zusammensetzung umfassend mindestens eine Verbindung nach Anspruch 1 als Wirkstoff zur Verwendung in der Schmerzbehandlung, wobei die Verbindung oder Zusammensetzung topisch an den entzündeten Bereich in einer therapeutisch wirksamen Menge verabreicht wird.

8. Verbindung oder Zusammensetzung zur Verwendung nach 7, wobei der Schmerz Kopfschmerz, Zahnschmerz und Muskelschmerz, sowie Arthritis- oder anderer Entzündungsschmerz ist.

9. Verbindung nach Anspruch 1 oder eine Zusammensetzung umfassend mindestens eine Verbindung nach Anspruch 1 als Wirkstoff zur Verwendung in der Behandlung von Akne, Sonnenbrand oder sonstigen Hautstörungen, und wobei die Verbindung oder Zusammensetzung in der Form einer Lösung, eines Sprays, einer Lotion, Salbe, Emulsion oder eines Gels ist.

10. Verbindungen der Struktur 1 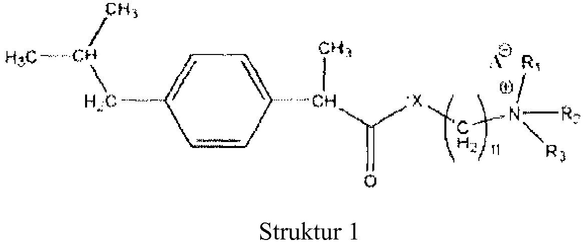 In der Struktur 1 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einem von jeden Alkyl-, Alkyloxy-, Alkenyl- oder Alkynyl-Resten mit bis zu 12 Kohlenstoffatomen, oder Aryl-Reste; R₃ entspricht H; X entspricht O oder S; A⁻ entspricht allen negativen Ionen; und n= 0, 1, 2, 3, 4, 5, 6, 7, 8, 9 oder 10, oder eine Zusammensetzung umfassend mindestens eine Verbindung der Struktur 1 als Wirkstoff zur Verwendung in der Behandlung von Akne, Sonnenbrand oder sonstigen Hautstörungen, und wobei die Verbindung oder Zusammensetzung in der Form einer Lösung, eines Sprays, einer Lotion, Salbe, Emulsion oder eines Gels ist, und wobei die Verbindung oder Zusammensetzung transdermal verabreicht wird.

11. Verbindung nach Anspruch 1 oder eine Zusammensetzung umfassend mindestens eine Verbindung nach Anspruch 1 als Wirkstoff zur Verwendung in der Behandlung von Asthma, wobei die Verbindung oder Zusammensetzung durch Sprühen in den Mund oder die Nase oder auf andere Körperteile verabreicht wird.

12. Verbindung nach Anspruch 1 oder eine Zusammensetzung umfassend mindestens eine Verbindung nach Anspruch 1 als Wirkstoff zur Verwendung in der Behandlung von Augenentzündungskrankheiten, Augenschmerzen nach Hornhautchirurgie, Glaukom- oder Ohrenentzündung und/oder schmerzhafte Zustände in Menschen oder Tieren.

13. Verbindung oder Zusammensetzung zur Verwendung nach Anspruch 12, wobei die Ohrenentzündung und/oder der schmerzhafte Zustand Otitis ist.

14. Transdermale therapeutische Anwendungssysteme von Verbindungen der Struktur 1 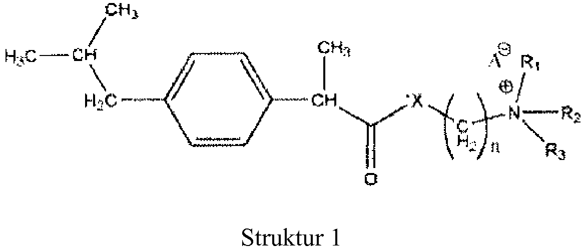 In der Struktur 1 entspricht R₁ H, einem von jeden Alkyl-, Alkyloxy-, Alkenyl- oder Alkynyl-Resten mit bis zu 12 Kohlenstoffatomen, oder Aryl-Reste; R₂ entspricht H, einen von jeden Alkyl-, Alkyloxy-, Alkenyl- oder Alkynyl-Resten mit bis zu 12 Kohlenstoffatomen, oder Aryl-Reste; R₃ entspricht H; X entspricht O oder S; A⁻ entspricht allen negativen Ionen; und n= 0, 1, 2, 3, 4, 5, 6, 7, 8, 9 oder 10, oder eine Zusammensetzung umfassend mindestens eine Verbindung der Struktur 1 als Wirkstoff zur Verwendung in der Behandlung von allen mit Ibuprofen behandelbaren Zuständen in Menschen oder Tieren, wobei transdermale therapeutische Anwendungssysteme dem Ibuprofen ermöglichen, konstant optimale Blutwerte zu erreichen, um die Wirksamkeit von Ibuprofen zu steigern und seine Nebenwirkungen zu reduzieren, wobei die mit Ibuprofen behandelbaren Zustände ausgewählt sind von der Gruppe einschließlich Zahnschmerzen, Kopfschmerzen, akute Migränekopfschmerzen, Schmerzen durch Arthritis, Entzündungsschmerzen, Dysmenorrhö, Fieber, Krebs, Bartter-Syndrom, vor und nach chronischer Uveitis, mit IUD verbundener uteriner Blutung, Übelkeit, durch Bestrahlung hervorgerufenes Erbrechen, diabetische Neuropathie, homophile Arthropathie, Knochenschwund und Sonnenbrand.

15. Transdermale therapeutische Anwendungssysteme nach Anspruch 14, **dadurch gekennzeichnet, dass** diese Systeme eine Bandage oder ein Pflaster sind, die eine Wirksubstanz enthaltende Matrixschicht und eine undurchlässige Trägerschicht beinhalten.

16. Transdermale therapeutische Anwendungssysteme nach den Ansprüchen 14-15, **dadurch gekennzeichnet, dass** das System einen Wirkstoffbehälter hat, der einen permeablen Boden gegenüber der Haut hat.

## Revendications

1. Composés de structure 1 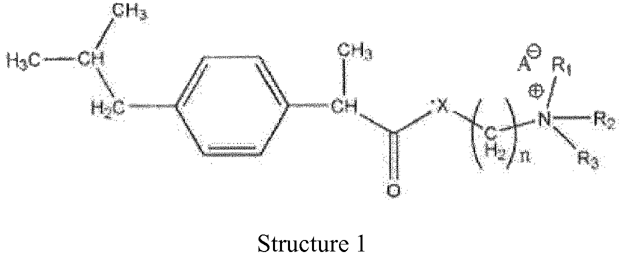 Dans la structure 1, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; R₃ représente un atome H ; X représente un atome S ; A⁻ représente des ions négatifs ; et n = 0, 1, 2, 3, 4, 5, 6, 7, 8, 9 ou 10.

2. Procédé de préparation de composés selon la revendication 1, des halogénures d'acides ou des anhydrides mixtes de structure 2 étant mis en réaction avec des composés de structure 3, dans lequel 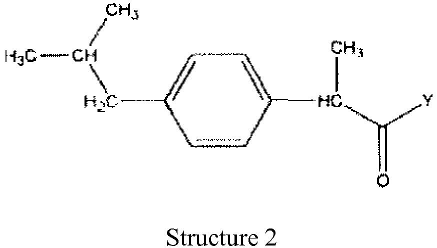 dans la structure 2, Y représente un atome d'halogène, un groupe alcoxycarbonyle ou aryloxy-carbonyloxy substitué, et dans lequel 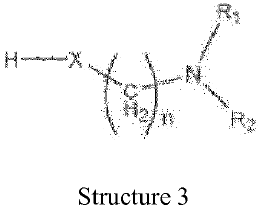 dans la structure 3, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; X représente un atome S ; et n = 0, 1, 2, 3, 4, 5, 6, 7, 8, 9 ou 10.

3. Procédé de préparation de composés selon la revendication 1, lesdits composés étant préparés à partir d'ibuprofène par réaction avec des composés de structure 3 au moyen de réactifs de couplage, dans lequel 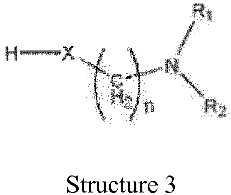 dans la structure 3, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; X représente un atome S ; et n = 0, 1, 2, 3, 4, 5, 6, 7, 8, 9 ou 10, et le réactif de couplage est choisi parmi le N,N'-dicyclohexylcarbodiimide, le N, N'-diisopropylcarbodiimide, le tétrafluoroborate de O-(benzotriazol-1-yl)-N,N,N',N'-tétraméthyluronium, l'hexafluorophosphate de O-(benzotriazol-1-yl)-N,N,N',N'-tétraméthyluronium et l'hexafluorophosphate de benzotriazol-1-yl-oxy-tris(diméthylamino)phosphonium.

4. Composé selon la revendication 1 ou composition comprenant au moins un composé selon la revendication 1 en tant que principe actif destiné à être utilisé dans le traitement des états traitables par l'ibuprofène chez l'homme et les animaux, ledit composé ou ladite composition étant administré par voie orale, lesdits états traitables par l'ibuprofène étant choisis dans le groupe comprenant une odontalgie, une céphalée, une céphalée migraineuse aiguë, une douleur arthritique, une douleur inflammatoire, une dysménorrhée, la fièvre, le cancer, le syndrome de Bartter, une uvéite chronique antérieure et postérieure, un saignement utérin associé à un DIU, des nausées, des vomissements induits par rayonnement, la neuropathie diabétique, l'arthropathie hémophilique, une perte osseuse et des coups de soleil.

5. Composés de structure 1 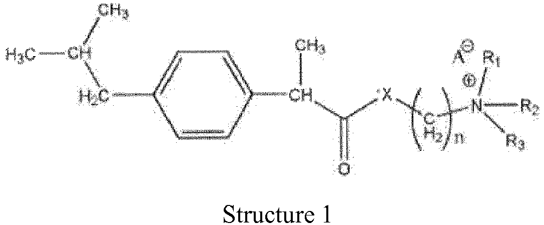 Dans la structure 1, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; R₃ représente un atome H ; X représente un atome O ou S ; A⁻ représente des ions négatifs ; et n = 0, 1, 2, 3, 4, 5, 6, 7, 8, 9 ou 10, ou composition comprenant au moins un composé de structure 1 en tant que principe actif destiné à être utilisé dans le traitement des états traitables par l'ibuprofène chez l'homme et les animaux, ledit composé ou ladite composition étant administré par voie transdermique, lesdits états traitables par l'ibuprofène étant choisis dans le groupe comprenant une odontalgie, une céphalée, une céphalée migraineuse aiguë, une douleur arthritique, une douleur inflammatoire, une dysménorrhée, la fièvre, le cancer, le syndrome de Bartter, une uvéite chronique antérieure et postérieure, un saignement utérin associé à un DIU, des nausées, des vomissements induits par rayonnement, la neuropathie diabétique, l'arthropathie hémophilique, une perte osseuse et des coups de soleil.

6. Composé ou composition destiné à être utilisé selon la revendication 5, ledit composé ou ladite composition étant utilisé sous la forme d'une solution, d'un spray, d'une lotion, d'un onguent, d'une émulsion ou d'un gel, et étant administré par voie transdermique dans toute partie du corps pour délivrer des niveaux plasmatiques thérapeutiquement efficaces.

7. Composé selon la revendication 1 ou composition comprenant au moins un composé selon la revendication 1 en tant que principe actif destiné à être utilisé dans le traitement d'une douleur, ledit composé ou ladite composition étant administré par voie topique dans la zone d'inflammation en quantité thérapeutiquement efficace.

8. Composé ou composition destiné à être utilisé selon la revendication 7, ladite douleur étant une céphalée, une odontalgie, une douleur musculaire, et une douleur arthritique et une autre douleur inflammatoire.

9. Composé selon la revendication 1 ou composition comprenant au moins un composé selon la revendication 1 en tant que principe actif destiné à être utilisé dans le traitement de l'acné, des coups de soleil ou d'autres troubles de la peau, et ledit composé ou ladite composition se présentant sous la forme d'une solution, d'un spray, d'une lotion, d'un onguent, d'une émulsion ou d'un gel.

10. Composés de structure 1 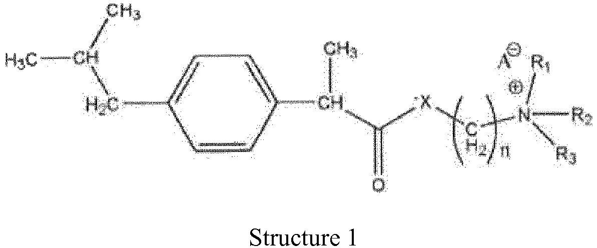 Dans la structure 1, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; R₃ représente un atome H ; X représente un atome O ou S ; A⁻ représente des ions négatifs ; et n = 0, 1, 2, 3, 4, 5, 6, 7, 8, 9 ou 10, ou composition comprenant au moins un composé de structure 1 en tant que principe actif destiné à être utilisé dans le traitement de l'acné, des coups de soleil ou d'autres troubles de la peau, ledit composé ou ladite composition se présentant sous la forme d'une solution, d'un spray, d'une lotion, d'un onguent, d'une émulsion ou d'un gel, et ledit composé ou ladite composition étant administré par voie transdermique.

11. Composé selon la revendication 1 ou composition comprenant au moins un composé selon la revendication 1 en tant que principe actif destiné à être utilisé dans le traitement de l'asthme, ledit composé ou ladite composition étant administré par pulvérisation à travers la bouche ou le nez ou d'autres parties du corps.

12. Composés selon la revendication 1 ou composition comprenant au moins un composé selon la revendication 1 en tant que principe actif destiné à être utilisé dans le traitement de maladies inflammatoires oculaires, d'une douleur oculaire après chirurgie de la cornée, un glaucome ou des affections inflammatoires et/ou douloureuses des oreilles chez l'homme ou les animaux.

13. Composé ou composition destiné à être utilisé selon la revendication 12, ladite affection inflammatoire et/ou douloureuse des oreilles étant une otite.

14. Systèmes d'application thérapeutiques transdermiques de composés de structure 1 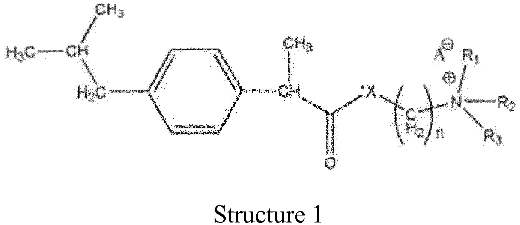 Dans la structure 1, R₁ représente un atome H, l'un de résidus alkyle, alkyloxy, alcényle ou alcynyle comportant jusqu'à 12 atomes de carbone, ou des résidus aryle ; R₂ représente un atome H, l'un de résidus alkyle, alkyloxy, alcényle ou alcynyle comportant jusqu'à 12 atomes de carbone, ou des résidus aryle ; R₃ représente un atome H ; X représente un atome O ou S ; A⁻ représente des ions négatifs ; et n = 0, 1, 2, 3, 4, 5, 6, 7, 8, 9 ou 10, ou composition comprenant au moins un composé de structure 1 en tant que principe actif destiné à être utilisé dans le traitement des états traitables par l'ibuprofène chez l'homme et les animaux, lesdits systèmes d'application thérapeutiques transdermiques permettant à l'ibuprofène d'atteindre des niveaux sanguins thérapeutiques constamment optimaux pour augmenter l'efficacité et réduire les effets secondaires de l'ibuprofène, lesdits états traitables par l'ibuprofène étant choisis dans le groupe comprenant une odontalgie, une céphalée, une céphalée migraineuse aiguë, une douleur arthritique, une douleur inflammatoire, une dysménorrhée, la fièvre, le cancer, le syndrome de Bartter, une uvéite chronique antérieure et postérieure, un saignement utérin associé à un DIU, des nausées, des vomissements induits par rayonnement, la neuropathie diabétique, l'arthropathie hémophilique, une perte osseuse et des coups de soleil.

15. Systèmes d'application thérapeutiques transdermiques selon la revendication 14, **caractérisés en ce que** ces systèmes sont un pansement ou un timbre dermique comprenant une couche matricielle contenant un principe actif et une couche de support imperméable.

16. Systèmes d'application thérapeutiques transdermiques selon les revendications 14 à 15, **caractérisés en ce que** le système comporte un réservoir de principe actif qui possède un fond perméable faisant face à la peau.
